# EUROPEAN PATENT SPECIFICATION

(11) **EP 2 134 326 B1**
(45) Date of publication and mention of the grant of the patent: **16.11.2011**
(21) Application number: 08709278.9
(22) Date of filing: 04.03.2008
(51) Int. Cl.: A61K 9/28, A61K 9/16, A61K 31/00

(54) **PHARMACEUTICAL COMPOSITIONS COMPRISING IRBESARTAN**
PHARMAZEUTISCHE ZUSAMMENSETZUNGEN ENTHALTEND IRBESARTAN
COMPOSITIONS PHARMACEUTIQUES CONTENANT DE L'IRBESARTAN

(30) Priority: 17.04.2007 IN DE08402007
(43) Date of publication of application: 23.12.2009
(73) Proprietor: Ratiopharm GmbH, 89079 Ulm (DE)
(72) Inventor: JEGANATHAN, Balamurugan, Madurai 625 011 (IN); GAT, Ganesh, V., Margoa 403 601 (IN); HUSSAIN, Jawed, Panaji 403 002 (IN)
(74) Representative: Kalhammer, Georg
(86) International application number: PCT/EP2008/052610
(87) International publication number: WO 2008/125388

(56) References cited:
- EP-A- 1 275 391
- WO-A-03/030868
- FR-A- 2 780 403

## Description

The present invention relates to a pharmaceutical composition comprising irbesartan, as disclosed in claim 1.

Irbesartan, 2-n-butyl-4-spirocyclopentane-1-[(2'-(tetrazol-5-yl)biphenyl-4-yl)methyl]-2-imidazolin-5-one, is a known angiotensin-II antagonist with the following chemical structure:

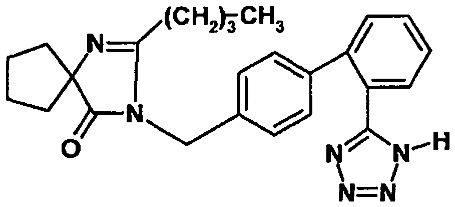

Irbesartan is particularly useful in the treatment of cardiovascular ailments such as hypertension and heart failure and is described in US patent no. 5,270,317.

WO-A-91114679 discloses the use of irbesartan for the treatment of hypertension and cardiac insufficiency.

Irbesartan exists in more than one polymorph forms. Polymorph forms A and B are disclosed in EP 0 708 103 and DE 695 19 788, respectively. WO 031050110 discloses an amorphous form of irbesartan.

Irbesartan has an acceptable oral bioavailability, however, a very unpleasant taste which renders the administration in dissolved form, such as in form of a syrup, disadvantageous. Therefore, a preferred form of administration is a tablet, which comprises a substantial amount of irbesartan as active ingredient. For the administration of irbesartan the dissolution profile of the tablet is a critical parameter. The tablet should provide a fast and complete release of the active ingredient.

However, irbesartan has some properties which make it difficult to formulate a large amount of the drug into a small tablet, for example it is a fluffy material with relative low bulk and tap densities and it has undesirable flow characteristics, such that it is sticky and can adhere to surfaces such as tablet punch faces and dies, causing problems in tabletting, especially on a high speed tablet press. The low aqueous solubility of irbesartan also presents a challenge, since, to keep the tablet mass small, only limited amounts of the excipients may be added to facilitate wetting, disintegration and finally, rapid and complete drug release. The addition of a diuretic, such as hydrochlorothiazide, which is also a fluffy material exhibiting poor flow and low aqueous solubility, can further contribute to tabletting problems. Such difficulties are disclosed in EP-A-747 050. According to this document a tablet should provide a dissolution profile such that 80% or more of the active ingredient irbesartan or a salt thereof contained in the tablet should be dissolved within 30 minutes.

To provide a tablet with such a dissolution profile, EP-A-747 050 suggests a tablet comprising irbesartan, a diluent, a binder, a lubricant, and in particular from about 1 to about 10 % of a disintegrant and from about 0.1 to about 5 % of an antiadherent. The antiadherent appears to be necessary in the disclosed formulations to avoid sticking of the active ingredient during tablettation.

EP03030868, FR2780403, EP1275391 disclose irbesartan-containing compositions. WO03030868 discloses a flash-melt pharmaceutical dosage form comprising a medicament, a superdisintegrant, a dispersing agent, a binder and further silicon dioxide.

In FR2780403 a composition with 20-50 % irbesartan, 2-20 % diuretic, 1-70 % diluent, 10-20 % binder, 4-6 % disintegrant, 0.5-1.0 % antiadherent (silicon dioxide) and 0.5-1.5 % lubricant is demonstrated.

Rep1275391 provides evidence for pharmaceutical compositions (tablet formulations) comprising 20-70 % irbesartan, 2-33 % of a diuretic, 1-70 % of a diluent, 2 % of a binder, a disintegrant, an antiadherent (silicon dioxide) and a lubricant.

There is still a need to provide improved fast dissolving pharmaceutical compositions containing irbesartan.

It has surprisingly been found that there is no need to add an antiadherent in a pharmaceutical formulation containing irbesartan, if greater amounts of disintegrants, in particular of "real" disintegrants, are used.

Therefore, the present application relates to a pharmaceutical composition comprising 30-80wt-% irbesartan or a pharmaceutically acceptable salt thereof as active ingredient, and at least 10 weight-% of a disintegrant, based on the total weight of the composition, characterized in that the composition does not comprise a silicon-containing antiadherent. Preferably, the composition comprises no antiadherent at all.

Preferably, the pharmaceutical composition according to the present invention comprises irbesartan or a pharmaceutically acceptable salt thereof in an amount of 30-80 weight-%, even more preferably 40-60 weight-%, in particular 50-60 weight-%, such as about 52 weight-% of irbesartan or a pharmaceutically acceptable salt thereof, based on the total weight of the composition.

The pharmaceutical composition of the present invention may contain more than 10 weight-%, preferably at least 10.5 weight-%, more preferably at least 11 weight-%, in particular at least 11.5 weight-%, e.g. about 11.7 weight-% or more of disintegrant, based on the total weight of the composition.

The pharmaceutical composition of the present invention preferably does not comprise a silicon-containing adherent, more preferably it does not comprise any antiadherent. An "antiadherent" according to the present invention is a compound which is capable of reducing the stickiness of the formulation, for example, preventing adherence to metal surfaces. Typically such antiadherents are silicon-containing compounds, such as silicon dioxide, magnesium trisilicate, or talc. In another embodiment, the composition of this invention comprises less than 0.1 weight-%, preferably less than 0.05 weight-%, most preferably less than 0.01 weight-% of antiadherent.

The pharmaceutical compositions of the present application contain irbesartan as active ingredient, which may be present in any polymorph or amorphous forms or a mixture of such forms, in particular in the polymorph form A or in the polymorph form B or a mixture thereof, optionally additionally in a mixture with amorphous form. Most preferably the irbesartan is present in polymorph form A, such as disclosed in EP 0 708 103, and DE 695 19 788, respectively.

The irbesartan as active ingredient may be present within the pharmaceutical compositions according to the present application in a mixture with a diuretic, such as e.g. described in EP-A 1 275 391, in particular with hydrochlorothiazide. For the case that such a diuretic is present in the pharmaceutical composition according to the present application, it is preferably present in an amount of 0.1 to 20 weight-% or preferably 1 to 10 weight-%, in particular 3 to 5 weight-%, e.g. about 4.2 weight%. In the presence of a diuretic the amount of irbesartan in the pharmaceutical composition of the present application is preferably the same as in the pharmaceutical compositions of the present application comprising irbesartan as the sole active ingredient, whereby the total amount of the combination of active ingredients is not more than 90 weight-%, in particular not more than 85 weight-% of the pharmaceutical composition of the present application.

The pharmaceutical composition of the present application comprises at least 10 weight-% of a disintegrant, based on the total weight of the composition. Some disintegrants are known in the art which may also be used as binders, fillers or as further excipients or adjuvants different to disintegrants. Such disintegrants having multiple functions are not disintegrants according to the present application, but are regarded as compounds of the other class they represent, i.e. binders, fillers, or other excipients or adjuvants different from disintegrants. The disintegrants according to the present application are therefore "real" disintegrants, and can only be used as disintegrants. Such a "real" disintegrant, which is the preferably used disintegrant according to the present application is croscarmellose sodium. Other disintegrants which are "real" disintegrants are known to the person skilled in the art and include, but are not limited to crospovidone, sodium starch glycolate or polacrilin potassium. Examples for components which are not real disintegrants and therefore not disintegrants according to the present application are for example microcrystalline cellulose which is a filler, or hydroxypropylcellulose which is a binder.

The pharmaceutical composition according to the present invention can further comprise additional excipients and adjuvants, which are pharmaceutically acceptable, and general coating materials, which are preferably applied as a coating to the solid form of the pharmaceutical composition of the present invention. Such further excipients and adjuvants are known to the person skilled in the art, and it can be referred to the standard textbook by Fiedler ("Lexikon der Hilfsstoffe für Pharmazie, Kosmetik und angrenzende Gebiete", 5th ed., 2002) or to the "Handbook of Excipients", edited by the American Pharmaceutical Association and Dr. Arthur H. Kibbe, 3rd ed., 2000.

The pharmaceutical composition according to the present application preferably comprises one or more fillers, binders, lubricants and/or coating materials and optionally colourants and/or surfactants, and in particular 0 to 30 weight-% of a filler, 0 to 10 weight-% of a binder, 0 to 3 weight-% of a lubricant, 0 to 3 weight-% of a coating material, and optionally 0 to 6 weight-% surfactant and 0 to 3 weight-% colourant based on the total weight of the composition. The further excipients and adjuvants and optionally coating materials or colourants are present in the pharmaceutical composition of the present application, such that the total amount of the pharmaceutical composition results in 100 weight %.

As filler one or more components can be used, which contribute a part of the tablet to reach the necessary total mass of the tablet. Preferable fillers are inorganic phosphates, like dibasic calcium phosphate, or sugars or sugar analogues and derivatives thereof, in particular lactose, such as lactose monohydrate or water-free lactose, dextrose, sorbit, mannit, saccharose, maltodextrin, isomalt and tablettose. Celluloses like microcrystalline cellulose or powdered celluloses are also preferable fillers according to the present application. Preferably the pharmaceutical composition according to the present application comprises 5 to 30 weight-%, more preferable 10 to 30 weight-%, even more preferable 15 to 25 weight-%, in particular 16 to 22 weight-%, e.g. about 20 weight-% of a filler.

As binders, i.e. a compound, enabling granulation of the active ingredient and the further excipients and adjuvants into granules, to be used in the pharmaceutical compositions of the present application, gelatin, povidone (N-vinylpropylidone polymer), copovidone (copolymer of N-vinyl-2-pyrrolidone and vinylacetate) and in particular hydroxypropylmethylcellulose can be exemplified, the latter being particularly preferred. The binder is usually present in an amount of 0 to 10 weight-%, preferably 2 to 8 weight-%, in particular 2 to 6 weight-%, based on the total weight of the composition.

As lubricants to be used in the pharmaceutical compositions of the present application fatty acids or fatty acid derivatives, such as alkali and earth alkali salts of stearic, lauric and/or palmitic acid, in particular glycerol monostearate or glycerol tristearate, glycerol palmic stearate, sodium lauryl stearate, sodium stearyl fumarate, zinc stearate, hydrogenated plant oil (lubritab), natriumbenzoate, or polyethylene glycol are exemplified, sodium stearyl fumarate and magnesium stearate are especially preferred. The lubricant is usually present in an amount of 0 - 3 weight-%, preferably 0.5 - 2.5 weight-%, such as about 2 weight-%, based on the total amount of the composition.

As surfactants or surface active agents, i.e. one or more compounds which are capable of improving the wetting of the tablets and/or enhancing the dissolution, to be used in the pharmaceutical compositions of the present application, sodium lauryl sulphate, polysorbates (e.g. Tween 80) and poloxamers are preferred. The surfactant is usually present in an amount of 0 - 6 weight-%, preferably 0 - 4 weight-%, in particular 0 - 2 weight-% based on the total amount of the composition.

In a preferred embodiment of the present application the pharmaceutical composition is in the form of a tablet which can be obtained by methods known in the art, such as tablettation by granulation or direct compression. Tablettation by granulation, in particular wet granulation is the preferred method to obtain the tablets of the present application. Preferably the tablet according to the present application has a dissolution profile such that at least 80 weight-% or more of the irbesartan compound contained in the tablet is dissolved within 30 minutes, in particular within 20 minutes.

The pharmaceutical composition according to the present invention comprising irbesartan may be present in form of a tablet which is coated with one or more coating materials. The coating materials are not particularly limited and are known to the person skilled in the art. As far as it is referred to a dissolution profile within this application, the dissolution profile is that of an uncoated tablet, if the tablet is not coated and refers to a coated tablet, if the tablet is coated.

The active ingredient of the pharmaceutical composition of the present application is preferably irbesartan, which is neutral or present as a pharmaceutically acceptable salt, and is more preferably irbesartan of the polymorph form A.

In the present application the term "dissolution profile" refers to the time course of the amount of irbesartan which is dissolved, relative to the total amount of irbesartan contained within the tablet. Such a dissolution profile can be obtained by dissolving a tablet in an USP-apparatus II in 1000 ml 0.1 N hydrochloric acid at 37 °C and a stirring speed of 50 rpm and the amount of irbesartan dissolved is measured over a defined period of time, e.g. 60 minutes, at distinct time points, e.g. every 5 minutes. The measurement of the irbesartan dissolved may be conducted by the detection of absorption of UV light, e.g. at 244 nm.

Preferably, the process for the preparation of the tablet of the present application is a wet granulation process, such that the active ingredients, i.e. irbesartan and optionally a diuretic, in particular hydrochlorothiazide, and a part of the filler, such as lactose or microcrystalline cellulose, and a part of the disintegrant, such as croscarmellose sodium, is mixed and optionally screened through a suitable mesh to break up any aggregates. The milled powder is further mixed, followed by granulation with a solution of the binder, such as an aqueous solution of hydroxypropylmethylcellulose (HPMC), in a suitable mixer/granulator. The wet granules can be dried, e.g. until a loss on drying (LOD) of 2.5 % or less is reached, followed by milling of the dried granules. The remaining excipients and adjuvants, such as the filler and the disintegrant can then be screened and mixed with the dried, milled granules in a mixer, followed by mixing with the lubricant, which can be screened before. This blend can then be compressed into tablets using a suitable tablet press and can then be optionally coated using a suitable coating material, e.g. Opadry.

In a preferred embodiment of the present application, the preferably solid pharmaceutical composition according to the present invention, which is in particular a tablet, contains 75 - 300 mg, e.g. 75, 150 or 300 mg irbesartan.

Within the present application, percentages given are referred to the weight, based on the total weight of the composition, if not indicated otherwise or obvious for the person skilled in the art. The following examples are not intended to be limiting:

### Example 1

Irbesartan/Hydrochlorothiazide tablet

| Ingredients | Function | mg/Tab | % w/w |
|---|---|---|---|
| *Intra Granular Material* | | | |
| Irbesartan | Active | 300.00 | 52.2 |
| Hydrochlorothiazide | Active | 25.00 | 4.3 |
| Lactose Monohydrate | Filler | 110.00 | 19.1 |
| Hypromellose (HPMC) | Binder | 20.00 | 3.5 |
| Ac-Di-Sol (Croscarmellose sodium) | Disintegrant | 35.00 | 6.1 |
| *Extra Granular Material* | | | |
| Ac-Di-Sol (Croscarmellose sodium) | Disintegrant | 32.00 | 5.6 |
| Microcrystalline Cellulose | Filler | 32.80 | 5.7 |
| Sodium Stearyl Fumarate | Lubricant | 5.20 | 0.9 |
| Opadry | Coasting material | 15.00 | 2.6 |
| | | | |
| | Total Weight | 575.00 | 100 |

### Preparation process:

Irbesartan or irbesartan/hydrochlorothiazide tablets were prepared using a wet granulation process:

The active ingredient (irbesartan and optionally hydrochlorothiazide), a part of the filler, i.e. lactose and/or microcrystalline cellulose, and a part of the disintegrant, i.e. croscarmellose sodium, were weighted out as indicated in the table. Optionally, the powder blend can be then screened through suitable mesh to break up any aggregates if present. The milled powder blend was then mixed, followed by granulation with the binder solution, i.e. aqueous solution of HPMC, in a mixer/granulator to form the "intra granular material". The wet granules were dried (in a fluid bed dryer) until a LOD (loss of drying) of typically 2.5 % or less was reached, followed by milling of the dried granules.

The remaining filler and the remaining disintegrant were weighted, screened, and mixed in a mixture with the dried, milled granules. In a final step, the lubricant, i.e. sodium stearyl fumarate, was weighted, screened and mixed with the above granule blend. This final blend was then compressed into tablets using a suitable tablet press and then coated with the coating material, i.e. Opadry.

### Example 2

Irbesartan/Hydrochlorothiazide tablet

| Ingredients | Function | mq/Tab | % w/w |
|---|---|---|---|
| *Intra Granular Material* | | | |
| Irbesartan | Active | 300.00 | 52.2 |
| Hydrochlorothiazide | Active | 25.00 | 4.3 |
| Microcrystalline Cellulose | Filler | 110.00 | 19.1 |
| Hypromellose (HPMC) | Binder | 20.00 | 3.5 |
| Explotab (Sodium Starch Glycolate) | Disintegrant | 35.00 | 6.1 |
| *Extra Granular Material* | | | |
| Explotab (Sodium Starch Glycolate) | Disintegrant | 32.00 | 5.6 |
| Microcrystalline Cellulose | Filler | 32.80 | 5.7 |
| Sodium Stearyl Fumarate | Lubricant | 5.20 | 0.9 |
| Opadry | Coating material | 15.00 | 2.6 |
| | | | |
| | Total Weight | 575.00 | 100 |

The tablet was prepared as described in Example 1.

### Example 3

Irbesartan/Hydrochlorothiazide tablet

| Ingredients | Function | mg/Tab | % w/w |
|---|---|---|---|
| *Intra Granular Material* | | | |
| Irbesartan | Active | 300.00 | 51.7 |
| Hydrochlorothiazide | Active | 25.00 | 4.3 |
| Lactose Monohydrate | Filler | 110.00 | 19.0 |
| Hypromellose (HPMC) | Binder | 30.00 | 5.2 |
| Crospovidone | Disintegrant | 35.00 | 6.0 |
| *Extra Granular Material* | | | |
| Ac-Di-Sol (Croscarmellose sodium) | Disintegrant | 32.00 | 5.5 |
| Microcrystalline Cellulose | Filler | 28.00 | 4.8 |
| Sodium Stearyl Fumarate | Lubricant | 5.00 | 0.9 |
| Opadry | Coating material | 15.00 | 2.6 |
| | | | |
| | Total Weight | 580.00 | 100.00 |

The tablet was prepared as described in Example 1.

### Example 4

Irbesartan/Hydrochlorothiazide tablet

| Ingredients | Function | mg/Tab | % w/w |
|---|---|---|---|
| *Intra Granular Material* | | | |
| Irbesartan | Active | 300.00 | 54.1 |
| Hydrochlorothiazide | Active | 12.50 | 2.3 |
| Lactose Monohydrate | Filler | 112.50 | 20.3 |
| Hypromellose (HPMC) | Binder | 20.00 | 3.6 |
| Ac-Di-Sol (Croscarmellose sodium) | Disintegrant | 20.00 | 3.6 |
| *Extra Granular* | | | |
| Ac-Di-Sol (Croscarmellose sodium) | Disintegrant | 41.00 | 7.4 |
| Microcrystalline Cellulose | Filler | 34.00 | 6.1 |
| Sodium stearyl Fumarate | Lubricant | 5.00 | 0.9 |
| Opadry | Coating material | 10.00 | 1.8 |
| | | | |
| | Total Weight | 555.00 | 100.0 |

The tablet was prepared as described in Example 1.

### Example 5

Irbesartan/Hydrochlorothiazide tablet

| Ingredients | Function | mq/Tab | % w/w |
|---|---|---|---|
| *Intra Granular Material* | | | |
| Irbesartan | Active | 300.00 | 53.1 |
| Hydrochlorothiazide | Active | 12.50 | 2.2 |
| Microcrystalline Cellulose | Filler | 86.50 | 15.3 |
| Hypromellose (HPMC) | Binder | 30.00 | 5.3 |
| Ac-Di-Sol (Croscarmellose sodium) | Disintegrant | 30.00 | 5.3 |
| *Extra Granular* | | | |
| Ac-Di-Sol (Croscarmellose sodium) | Disintegrant | 36.00 | 6.4 |
| Microcrystalline Cellulose | Filler | 53.00 | 9.4 |
| Sodium Stearyl Fumarate | Lubricant | 7.00 | 1.2 |
| Opadry | Coating material | 10.00 | 1.8 |
| | | | |
| | Total Weight | 565.00 | 100.0 |

The tablet was prepared as described in Example 1.

### Example 6

Irbesartan/Hydrochlorothiazide tablet

| Ingredients | Function mg/Tab | % w/w |
|---|---|---|
| *Infra Granular Material* | | |
| Irbesartan | Active 300.00 | 52.2 |
| Hydrochlorothiazide | Active 12.50 | 2.2 |
| Lactose Monohydrate | Filler 97.50 | 17.0 |
| Hypromellose (HPMC) | Binder 30.00 | 5.2 |
| Ac-Di-Sol (Croscarmellose sodium) | Disintegrant 35.00 | 6.1 |
| *Extra Granular* | | |
| Ac-Di-Sol (Croscarmellose sodium) | Disintegrant 32.00 | 5.6 |
| Microcrystalline Cellulose | Filler 28.00 | 4.9 |
| Hydroxy Propyl cellulose (HPC-LH) | Binder 20.00 | 3.5 |
| Sodium Stearyl Fumarate | Lubricant 5.00 | 0.9 |
| Opadry | Coating material 15.00 | 2.6 |
| | | |
| | Total Weight 575.00 | 100.0 |

The tablet was prepared as described in Example 1.

### Example 7

Irbesartan tablet

| Ingredients | Function | mg/Tab | % w/w |
|---|---|---|---|
| *Intra Granular Material* | | | |
| Irbesartan | Active | 300.00 | 55.6 |
| Lactose Monohydrate | Filler | 110.00 | 20.4 |
| Hypromellose (HPMC) | Binder | 20.00 | 3.7 |
| Ac-Di-Sol (Croscarmellose sodium) | Disintegrant | 20.00 | 3.7 |
| *Extra Granular* | | | |
| Ac-Di-Sol (Croscarmellose sodium) | Disintegrant | 41.00 | 7.6 |
| Microcrystalline Cellulose | Filler | 34.00 | 6.3 |
| Sodium Stearyl Fumarate | Lubricant | 5.00 | 0.9 |
| Opadry | Coating material | 10.00 | 1.9 |
| | | | |
| | Total Weight | 540.00 | 100.0 |

The tablet was prepared as described in Example 1.

### Example 8

Irbesartan tablet

| Ingredients | Function | mg/Tab | % w/w |
|---|---|---|---|
| *Intra Granular Material* | | | |
| Irbesartan | Active | 300.00 | 53.1 |
| Microcrystalline Cellulose | Filler | 99.00 | 17.5 |
| Hypromellose (HPMC) | Binder | 30.00 | 5.3 |
| Ac-Di-Sol (Croscarmellose sodium) | Disintegrant | 30.00 | 5.3 |
| *Extra Granular* | | | |
| Ac-Di-Sol (Croscarmellose sodium) | Disintegrant | 36.00 | 6.4 |
| Microcrystalline Cellulose | Filler | 53.00 | 9.4 |
| Sodium Stearyl Fumarate | Lubricant | 7.00 | 1.2 |
| Opadry | Coating material | 10.00 | 1.8 |
| | | | |
| | Total Weight | 565.00 | 100.0 |

The tablet was prepared as described in Example 1.

### Example 9

Irbesartan tablet

| Ingredients | Function | mg/Tab % w/w |
|---|---|---|
| *Intra Granular Material* | | |
| Irbesartan | Active | 300.00 52.2 |
| Lactose Monohydrate | Filler | 110.00 19.1 |
| Hypromellose (HPMC) | Binder | 30.00 5.2 |
| Ac-Di-Sol (Croscarmellose sodium) | Disintegrant | 35.00 6.1 |
| *Extra Granular* | | |
| Ac-Di-Sol (Croscarmellose sodium) | Disintegrant | 32.00 5.6 |
| Microcrystalline Cellulose | Filler | 28.00 4.9 |
| Hydroxy Propyl cellulose (HPC-LH) | Binder | 20.00 3.5 |
| Sodium Stearyl Fumarate | Lubricant | 5.00 0.9 |
| Opadry | Coating material | 15.00 2.6 |
| | | |
| | Total Weight | 575.00 100.0 |

The tablet was prepared as described in Example 1.

### Example 10

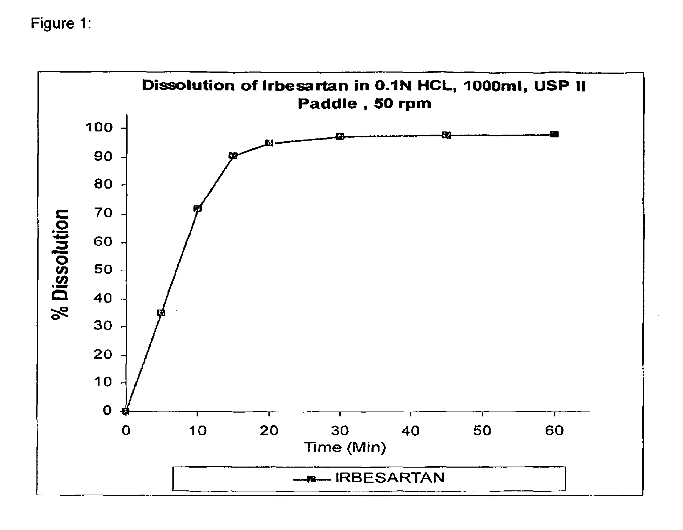

Tablets prepared according to example 4 were analyzed. The dissolution profile was obtained by dissolving a tablet in an USP-apparatus II in 1000 ml 0.1 N hydrochloric acid at 37 °C and a stirring speed of 50 rpm. The dissolution profile obtained is depicted in Figure 1.

## Claims

1. A pharmaceutical composition comprising 30 - 80 wt.-% of irbesartan or a pharmaceutically acceptable salt thereof as active ingredient and more than 10 wt% of disintegrant, based on the total weight of the composition, **characterized in that** the composition does not comprise a silicon-containing antiadherent.

2. The pharmaceutical composition according to claim 1, **characterized in that** it does not comprise an antiadherent.

3. The pharmaceutical composition according to claim 1 or 2 which contains 40 - 60 wt% of irbesartan, based on the total weight of the composition.

4. The pharmaceutical composition according to any one of the preceding claims, **characterized in that** the disintegrant is one or more compound selected from croscarmellose sodium, crospovidone, sodium starch glycolate and polacrilin potassium.

5. The pharmaceutical composition according to any one of the preceding claims which comprises a diuretic as further active ingredient.

6. The pharmaceutical composition according to claim 5, wherein the diuretic is hydrochlorothiazide.

7. The pharmaceutical composition according to any one of the preceding claims which further comprises one or more excipients or adjuvants, selected from the group consisting of fillers, binders, lubricants, surfactants, colourants and coating materials.

8. The pharmaceutical composition according to claim 7 which comprises:
0 - 30 wt% of a filler,
0 -10 wt% of a binder
0 - 3 wt% of a lubricant,
0 - 6 wt% of a surfactant,
0 - 3 wt% of a colourant, and
0 - 3 wt% of a coating material,
each based on that total weight of the composition.

9. The pharmaceutical composition according to any of the preceding claims which is in the form of a tablet.

10. The pharmaceutical composition according to claim 9, wherein the tablet is film coated.

11. The pharmaceutical composition according to claim 9 or 10, **characterized in that** it shows a dissolution profile such that 80 wt% or more of the irbesartan compound contained in the tablet is dissolved within 30 minutes.

12. Process for the preparation of a pharmaceutical composition according to claims 9 to 11, comprising mixing the irbesartan or a pharmaceutically acceptable salt thereof with a part of the excipients and adjuvants, wet granulation of the mixture, mixing the granules with the remaining excipients and adjuvants, drying the mixture, preparing tablets from said mixture and optionally film-coating said tablets.

## Patentansprüche

1. Pharmazeutische Zusammensetzung, umfassend 30 - 80 Gew.-% Irbesartan oder ein pharmazeutisch akzeptables Salz davon als Wirkstoff und mehr als 10 Gew.-% Desintegrationsmittel, basierend auf dem Gesamtgewicht der Zusammensetzung, **dadurch gekennzeichnet, dass** die Zusammensetzung kein siliciumhaltiges Antihaftmittel umfasst.

2. Pharmazeutische Zusammensetzung nach Anspruch 1, **dadurch gekennzeichnet, dass** sie kein Antihaftmittel umfasst.

3. Pharmazeutische Zusammensetzung nach Anspruch 1 oder 2, welche 40 - 60 Gew.-% Irbesartan, basierend auf dem Gesamtgewicht der Zusammensetzung, umfasst.

4. Pharmazeutische Zusammensetzung nach einem der vorstehenden Ansprüche, **dadurch gekennzeichnet, dass** das Desintegrationsmittel eine oder mehrere Verbindung(en), ausgewählt aus Croscarmellose-Natrium, Crospovidon, Natriumstärkeglycolat und Polacrilinkalium, ist.

5. Pharmazeutische Zusammensetzung nach einem der vorstehenden Ansprüche, welche ein Diuretikum als weiteren Wirkstoff umfasst.

6. Pharmazeutische Zusammensetzung nach Anspruch 5, wobei das Diuretikum Hydrochlorthiazid ist.

7. Pharmazeutische Zusammensetzung nach einem der vorstehenden Ansprüche, welche ferner einen oder mehrere Hilfsstoffe oder Adjuvanzien, ausgewählt aus der Gruppe, bestehend aus Füllstoffen, Bindemitteln, Schmiermitteln, oberflächenaktiven Mitteln, Farbstoffen und Beschichtungsmaterialien, umfasst.

8. Pharmazeutische Zusammensetzung nach Anspruch 7, welche:
0 - 30 Gew.-% eines Füllstoffs,
0 - 10 Gew.-% eines Bindemittels,
0 - 3 Gew.-% eines Schmiermittels,
0 - 6 Gew.-% eines oberflächenaktiven Mittels,
0 - 3 Gew.-% eines Farbstoffes und
0 - 3 Gew.-% eines Beschichtungsmaterials,
jeweils basierend auf dem Gesamtgewicht der Zusammensetzung, umfasst.

9. Pharmazeutische Zusammensetzung nach einem der vorstehenden Ansprüche in Form einer Tablette.

10. Pharmazeutische Zusammensetzung nach Anspruch 9, wobei die Tablette filmbeschichtet ist.

11. Pharmazeutische Zusammensetzung nach Anspruch 9 oder 10, **dadurch gekennzeichnet, dass** sie ein solches Auflösungsprofil zeigt, dass 80 Gew.-% oder mehr der Irbesartan-Verbindung, die in der Tablette enthalten ist, innerhalb von 30 Minuten gelöst werden.

12. Verfahren zur Herstellung einer pharmazeutischen Zusammensetzung nach den Ansprüchen 9 bis 11, umfassend das Mischen von Irbesartan oder eines pharmazeutisch akzeptablen Salzes davon mit einem Teil der Hilfsstoffe und Adjuvanzien, Nassgranulation des Gemisches, Mischen der Granulate mit den verbleibenden Hilfsstoffen und Adjuvanzien, Trocknen des Gemisches, Herstellen von Tabletten aus dem Gemisch und gegebenenfalls Filmbeschichten der Tabletten.

## Revendications

1. Composition pharmaceutique comprenant 30 à 80 % en poids d'irbesartan ou un de ses sels pharmaceutiquement acceptable comme ingrédient actif et plus de 10 % en poids d'agent désintégrant, en se basant sur le poids total de la composition, **caractérisée en ce que** la composition ne comprend pas d'antiadhésif à base de silicone.

2. Composition pharmaceutique selon la revendication 1, **caractérisée en ce qu'**elle ne comprend pas d'antiadhésif.

3. Composition pharmaceutique selon la revendication 1 ou 2, qui contient 40 à 60 % en poids d'irbesartan, en se basant sur le poids total de la composition.

4. Composition pharmaceutique selon l'une quelconque des revendications précédentes, **caractérisée en ce que** l'agent désintégrant est formé d'un ou plusieurs composés sélectionnés parmi la croscarmellose sodique, la crospovidone, le glycolate d'amidon sodique et la polacriline potassique.

5. Composition pharmaceutique selon l'une quelconque des revendications précédentes, qui comprend un diurétique comme autre ingrédient actif.

6. Composition pharmaceutique selon la revendication 5, dans laquelle le diurétique est un hydrochlorothiazide.

7. Composition pharmaceutique selon l'une quelconque des revendications précédentes, qui comprend en outre un ou plusieurs excipients ou adjuvants, sélectionnés dans le groupe constitué des charges, des liants, des lubrifiants, des tensioactifs, des colorants et des matériaux d'enrobage.

8. Composition pharmaceutique selon la revendication 7, qui comprend :
0 à 30 % en poids d'une charge,
0 à 10 % en poids d'un liant,
0 à 3 % en poids d'un lubrifiant,
0 à 6 % en poids d'un tensioactif,
0 à 3 % en poids d'un colorant et
0 à 3 % en poids d'un matériau d'enrobage,
chaque proportion se basant sur le poids total de la composition.

9. Composition pharmaceutique selon l'une quelconque des revendications précédentes, qui se présente sous la forme d'un comprimé.

10. Composition pharmaceutique selon la revendication 9, dans laquelle le comprimé est pelliculé.

11. Composition pharmaceutique selon la revendication 9 ou 10, **caractérisée en ce qu'**elle présente un profil de dissolution tel que 80 % en poids ou plus du composé irbesartan contenu dans le comprimé soient dissous en 30 minutes.

12. Procédé pour la préparation d'une composition pharmaceutique selon les revendications 9 à 11, comprenant un mélange de l'irbesartan ou d'un de ses sels pharmaceutiquement acceptable avec une partie des excipients et des adjuvants, une granulation par voie humide du mélange, un mélange des granulés avec les excipients et les adjuvants restants, un séchage du mélange, une préparation de comprimés à partir dudit mélange et éventuellement un pelliculage desdits comprimés.
